Europäisches Patentamt

**European Patent Office**

**Office européen des brevets**

(11) Publication number: **0 136 019**
A2

# EUROPEAN PATENT APPLICATION

(21) Application number: **84305438.8**

(22) Date of filing: **09.08.84**

(51) Int. Cl.⁴: **A 61 F 5/00,** A 61 F 5/44

(30) Priority: **19.08.83 GB 8322447**

(43) Date of publication of application: **03.04.85**
**Bulletin 85/14**

(84) Designated Contracting States: **AT BE CH DE FR GB IT LI LU NL SE**

(71) Applicant: **CRAIG MEDICAL PRODUCTS LIMITED, Blackfriars House 19 New Bridge Street, London, EC4 6BY (GB)**

(72) Inventor: **Steer, Peter Leslie, Woodlands Rise, Kingscote East Grinstead Sussex (GB)**

(74) Representative: **Cook, Anthony John et al, D. YOUNG & CO. 10, Staple Inn, London, WC1V 7RD (GB)**

(54) **Drainage bag.**

(57) The invention relates to a drainage bag primarily for urine but also for other liquid drainage, and particularly although not exclusively to a urostomy bag.

There has long been a problem in draining urine bags. Users who are not dexterous, and particularly those who are old or infirm, find it difficult to satisfactorily manipulate present bag-emptying arrangements when emptying the bag. Present emptying arrangements involve a plug and tube, or a folding tube with a clip, or a valve or tap. It is often difficult to aim the bag or tap (10) outlet directly into the eventual urine receptacle.

These problems are largely or wholly overcome by providing a siphon tube for emptying a drainage bag.

## DRAINAGE BAG

This invention relates to a drainage bag primarily for urine but also for other liquid drainage, and particularly althought not exclusively to a urostomy bag.

There has long been a problem in draining urine bags. Users who are not dexterous, and particularly those who are old or infirm, find it difficult to satisfactorily manipulate present bag-emptying arrangements when emptying the bag. Present emptying arrangements involve a plug and tube, or a folding tube with a clip, or a valve or tap. The bag or tap outlet must be aimed directly into the eventual urine receptacle, and it is easy for a non-dexterous person when manipulating a tap or a folding tube or a plugged tube to allow urine to escape prematurely or inaccurately, with uncomfortable or unpleasant results.

According to the present invention, a siphon tube is provided for emptying a drainage bag.

In a preferred form of the invention, the siphon tube is located largely within the bag and one open end of the tube is located in a lower region of the bag with a loop, the tube extending internally of the bag, and the outlet end portion of the tube passing through a lower part of the bag wall and terminating in an outlet orifice at its other end.

The bag may be made of two superposed plastic sheets welded around their periphery and the outlet end of the tube may pass between them through a suitable gap in the weld, there being a suitable seal to prevent leakage where the tube exits the bag.

In use, a user whose bag is full or substantially full of urine aims the outlet tube, holding it in one hand, directly at the eventual receptacle, e.g., a lavatory bowl. He or she then presses the bag gently with the other hand so forcing the liquid in the bag into the open end of the siphon tube and upwardly in that tube until it passes the highest point, whereupon the bag empties steadily because of the siphon effect. No manipulation of a valve or tap is needed, there is no plug or clip to be unfitted or refitted, and no plug or clip to get lost. Bag emptying is converted from being an awkward, distasteful and possibly messy operation to being a simple task even for those who are now well coordinated and dexterous.

Non-limited examples of the invention will now be described with reference to the accompanying drawings in which:

Figure 1 is a front view of one example of urostomy bag according to the invention;

Figure 2 is a cross sectional side view of the bag of Figure 1 along the major axis; and

Figure 3 shows a second example of the invention.

The illustrated urostomy bag has a front wall 10 and a rear wall 12 joined by a peripheral join 14.  Usually the walls are of a plastic material joined by a peripheral weld 14.  The rear wall 12 has an orifice 16 for the stoma of the wearer, and the orifice 16 is surrounded by a coupling 18 by which the bag can be fixed to a pad or dressing worn by (e.g., adhesively fixed to) the wearer.  One suitable coupling is that described and claimed in British Patent No. 1,571,657.

A siphon tube 20 is located in the bag with one open end 22 (the liquid entry end) located in a lower region internally of the bag and an open outlet orifice 24 located outside the bag.  The outlet end portion 26 of the tube passes between the front and rear walls 10, 12 through a suitable gap or break in the weld seam.

In use, a full bag does not empty until the siphoning effect is established by squashing the bag gently, so forcing the liquid level in the tube 20 up to the point 28 in the tube whereupon it runs to the open end 24 and completely empties the bag automatically.  In this way, a bag may be easily emptied with no delicate manipulation while the user holds that portion of the tube between 26 and 24 and directs it accurately as necessary.

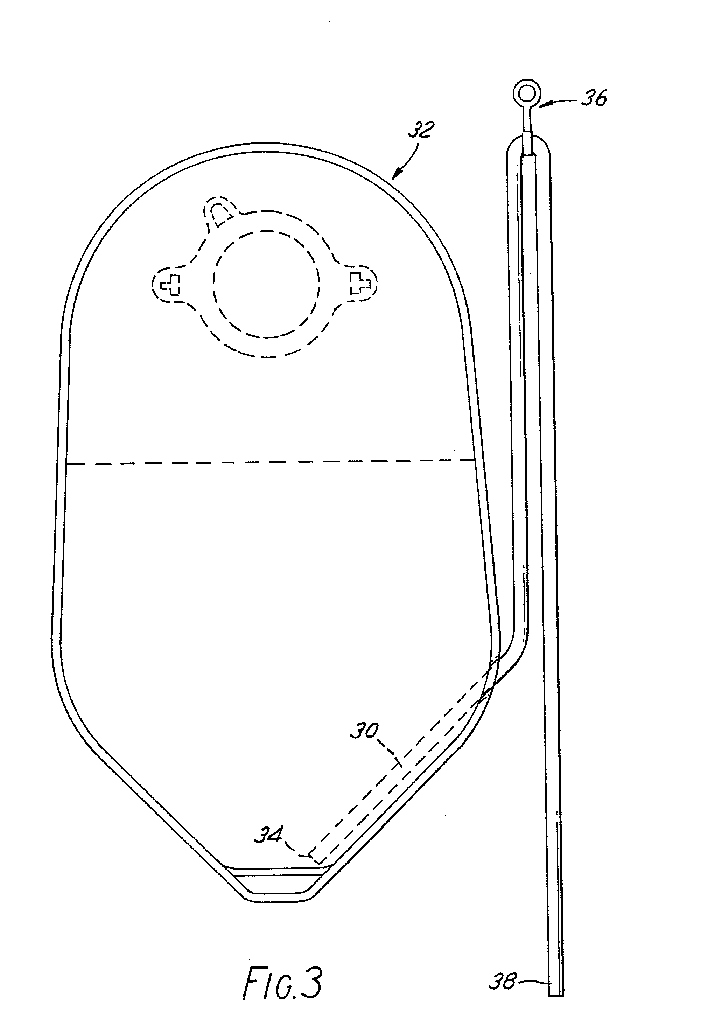

It will be appreciated that while the major part of the tube has been illustrated within the bag, this is not essential.  As seen for example in Figure 3, a siphon tube 30 could have a short part of its length located within a drainage bag 32, and an open inlet end 34 located in a lower region of the bag. The tube passes through the bag wall and a major part of its length is located outside the bag. A hook or clip 36 is provided so that the tube 30 is suitably suspended. Its outlet orifice is shown at 38. As a further alternative within the invention, the upwardly extending portion of the tube could be within the bag and the downwardly extending portion outside, possibly attached to the bag temporarily or permanently by a loop or sleeve on the bag front wall.

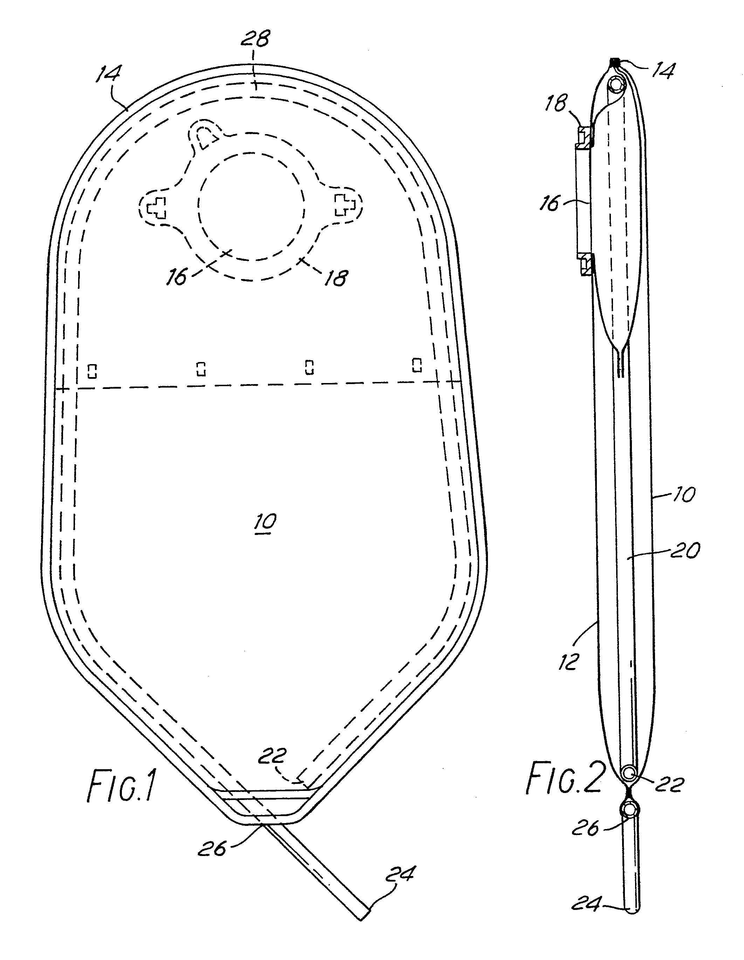

In an alternative embodment of the invention, the siphon tube could have a horizontal portion partly within the bag with a U-shaped portion (inverted U) extending upwardly from the horizontal portion and then downwardly to a lower level. Such an arrangement would be the functional equivalent of the arrangements illustrated in Figures 1 and 3.

The words "siphon tube" when used in this specification mean a pipe or tube shaped like an inverted "V" or "U" with unequal legs for conveying liquid and delivering it at a lower level by utilizing atmospheric pressure; and "siphon" when used as a verb has a corresponding meaning.

0136019

What we claim is:

1. A drainage bag including a siphon tube whereby it can be substantially emptied.

2. A bag according to claim 1 in which the siphon tube is located largely within the bag and one open end of the tube is located in a lower region of the bag with a loop, the tube extending internally of the bag, and the outlet end portion of the tube passing through a lower part of the bag wall and terminating in an outlet orifice at its other end.

3. A bag according to claim 1 in which the bag is made of two superposed plastic sheets welded around their periphery and the outlet end of the tube passes between them through a suitable gap in the weld, there being a suitable seal to prevent leakage where the tube exits the bag.

4. A bag according to claim 2 in which the bag is made of two superposed plastic sheets welded around their periphery and the outlet end of the tube passes between them through a suitable gap in the weld, there being a suitable seal to prevent leakage where the tube exits the bag.

5. A bag according to claim 1 in which the siphon tube is located largely outside the bag.

6. A bag according to claim 3 in which the siphon tube is located largely outside the bag.

7. A bag according to claim 5 which includes suspension means whereby an upper portion of the tube outside the bag can be suitably suspended.

8. A bag according to claim 6 which includes suspension means whereby an upper portion of the tube outside the bag can be suitably suspended.

9. A bag according to claim 2 in which a portion of the siphon tube is removably retained by a hook or sleeve carried by the bag.

10. A bag according to claim 4 in which a portion of the siphon tube is removably retained by a hook or sleeve carried by the bag.

11. A bag according to claim 5 in which a portion of the siphon tube is removably retained by a hook or sleeve carried by the bag.

12. A bag according to claim 9 in which the hook or sleeve is located on the bag front wall.

13. A bag according to claim 10 in which the hook or sleeve is located on the bag front wall.

14. A bag according to claim 11 in which the hook or sleeve is located on the bag front wall.

0136019

FIG.1

FIG.2

36

32

30

34

FIG.3

38